(19) Europäisches Patentamt
European Patent Office
Office européen des brevets

(11) EP 1 621 202 B1

(12) EUROPEAN PATENT SPECIFICATION

(45) Date of publication and mention of the grant of the patent:
**29.12.2010 Bulletin 2010/52**

(21) Application number: **04731472.9**

(22) Date of filing: **06.05.2004**

(51) Int Cl.:
***A61K 31/714*** (2006.01) ***A61K 47/10*** (2006.01)
***A61K 47/26*** (2006.01) ***A61P 25/00*** (2006.01)

(86) International application number:
**PCT/JP2004/005973**

(87) International publication number:
**WO 2004/098614 (18.11.2004 Gazette 2004/47)**

# (54) FREEZE-DRIED PREPARATION CONTAINING METHYLCOBALAMIN AND PROCESS FOR PRODUCING THE SAME

GEFRIERGETROCKNETES PRÄPARAT MIT METHYLCOBALAMIN UND VERFAHREN ZU SEINER HERSTELLUNG

PREPARATION LYOPHILISEE CONTENANT DE LA METHYLCOBALAMINE ET PROCEDE DE PRODUCTION DE CETTE PREPARATION

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **07.05.2003 JP 2003129251**

(43) Date of publication of application:
**01.02.2006 Bulletin 2006/05**

(73) Proprietor: **Eisai R&D Management Co., Ltd.**
**Tokyo 112-8088 (JP)**

(72) Inventors:
- **KATO, Akira**
**c/o Kawashima Industrial Park**
**Kagamigahara-shi, Gifu 501-6024 (JP)**
- **NOMURA, Teruko**
**c/o Kawashima Industrial Park**
**Kagamigahara-shi, Gifu 501-6024 (JP)**

(74) Representative: **Baldock, Sharon Claire**
**Boult Wade Tennant**
**Verulam Gardens**
**70 Gray's Inn Road**
**London WC1X 8BT (GB)**

(56) References cited:
**GB-A- 1 178 984** **JP-A- 9 136 837**
**JP-A- 10 218 775** **JP-A- 55 113 721**
**JP-A- 59 152 327** **US-A- 5 964 224**

- **LUECKEL B ET AL: "STRATEGY FOR OPTIMIZING THE LYOPHILIZATION OF BIOTECHNOLOGICAL PRODUCTS" PHARMACEUTICAL SCIENCES, LONDON, GB, vol. 3, no. 1, 1997, pages 3-8, XP000938579 ISSN: 1356-6881**
- **TELANG CHITRA ET AL: "Effective inhibition of mannitol crystallization in frozen solutions by sodium chloride." PHARMACEUTICAL RESEARCH (DORDRECHT), vol. 20, no. 4, April 2003 (2003-04), pages 660-667, XP009089462 ISSN: 0724-8741**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 621 202 B1

## Description

Technical Field

The present invention relates to a freeze-dried preparation for high-concentration injection containing methylcobalamin as a therapeutic drug for amyotrophic lateral sclerosis, and a process or producing the freeze-dried preparation.

Background of Art

Amyotrophic lateral sclerosis, also known as ALS, is a disease characterized mainly by lesions of the brain stem, spinal cord and motor neurons which causes progressive bulbar syndrome and disorders of the upper and lower motor neurons, and is an intractable disease in which degeneration and elimination of motor nerve cells of the spinal cord and cerebrum leads clinically to systemic amyotrophy and spasticity, thereby generally resulting in death due to paralysis of the respiratory muscles within 2 to 3 years.

The causes of amyotrophic lateral sclerosis are unknown, and currently there are not only no fundamental therapies but also no effective therapies to arrest or delay the progress of the disease.

In recent years it has been observed by Prof. Kaji that when administered large amounts of methylcobalamin (for example, methylcobalamin 15 to 50 mg/once daily via intramuscular injection), which is effective for treating peripheral nerve disorders in humans, to patients with amyotrophic lateral sclerosis exhibit dose-dependent improvement in subjective muscle strength and a significant increase in an objective marker, magnitude of compound muscle action potential. With the discovery that dramatic therapeutic effects are obtained which have not been achieved with conventional therapies, an aqueous injection for high-dose methylcobalamin preparations was proposed as a therapeutic drug for amyotrophic lateral sclerosis, which contain methylcobalamin as an active ingredient (see for example Japanese Patent Application Laid-open No. H10-218775).

Note that the dosage of methylcobalamin for treatment of peripheral nerve disorders in humans is generally from 0.5 to 1.5 mg/day.

However, it has been pointed out that because this aqueous injection is a solution of from 15 to 50 ml per ampoule of methylcobalamin dissolved in ethanol, saline or a buffer, there are the problems that it is difficult to ensure photostability and storage stability, and difficult to guarantee sterility.

On the other hand, methylcobalamin is a vitamin, and most research into freeze-dried preparations for injection containing methylcobalamin as an active ingredient has involved multi-vitamin injection preparations containing a variety of essential vitamins. In the case of these multi-vitamin preparations, the problems include ensuring stability during storage when the preparation is freeze-dried and improving a dissolved state after the drug is re-dissolved (see for example, Japanese Patent Applications Laid-Open Nos. S62-38, S63-3137 and H1-132514).

Disclosure of the Invention

Problems to be Solved by the Invention

In view of the above, it is the first object of the present invention to provide a freeze-dried preparation comprising a high content of methylcobalamin which has excellent stability over time and can be used in high-concentration methylcobalamin therapy.

Moreover, it is the second object of the present invention to provide a process for producing a freeze-dried preparation comprising methylcobalamin wherein methylcobalamin is stable in a freeze-dried solid.

Means for Solving the Problems

The inventors have intensively carried out the research aimed at achieving the aforementioned objects. As a result, they have discovered that methylcobalamin is more stable in an amorphous freeze-dried solid than in a crystalline freeze-dried solid, thereby arriving at the present invention.

That is, in the first aspect of the present invention, the first object is achieved by a freeze-dried preparation comprising methylcobalamin or a pharmacologically acceptable salt thereof and an excipient, wherein at least one or more of species of the excipient is in an amorphous state.

In a preferred aspect of the freeze-dried preparation according to the present invention, the methylcobalamin or pharmacologically acceptable salt thereof is also in an amorphous state.

In a preferred aspect of the freeze-dried preparation according to the present invention, the excipient which is in the amorphous state comprises a sugar.

In a preferred aspect of the freeze-dried preparation according to the present invention, the freeze-dried preparation further comprises a pH adjuster.

In a preferred aspect of the freeze-dried preparation according to the present invention, the freeze-dried preparation further comprises an anti-oxidant.

In a preferred aspect of the freeze-dried preparation according to the present invention, when the excipient comprises the sugar, the excipient in the amorphous state is contained in an amount of at least 20% by weight, based on the total weight of the excipient.

In a preferred aspect of the freeze-dried preparation according to the present invention, the sugar comprises sucrose or lactose.

Moreover, in the second aspect of the present invention, the first object is also achieved by a freeze-dried preparation comprising methylcobalamin or a pharmacologically acceptable salt thereof and an excipient, which is obtained by a production process which comprises the steps of: (a) dissolving the methylcobalamin or the pharmacologically acceptable salt thereof and the excipient in a solvent; and (b) freeze-drying the solution so as to produce an amorphous state of at least one or more species of the excipient.

In a preferred aspect of the freeze-dried preparation according to the present invention, freeze-drying in the step (b) is accomplished so as to further produce an amorphous state of the methylcobalamin or the pharmacologically acceptable salt thereof.

In a preferred aspect of the freeze-dried preparation according to the present invention, pre-freezing is performed in the step (b) at a temperature at which the excipient does not crystallize, or below the temperature. In particular, pre-freezing during the freeze-drying process is preferably at a temperature at or below an eutectic point of the excipient.

In a preferred aspect of the freeze-dried preparation according to the present invention, the excipient which is in the amorphous state comprises a sugar.

In a preferred aspect of the freeze-dried preparation according to the present invention, a pH adjuster is also dissolved in the step (a).

In a preferred aspect of the freeze-dried preparation according to the present invention, an anti-oxidant is also dissolved in the step (a).

In a preferred aspect of the freeze-dried preparation according to the present invention, when the excipient comprises the sugar, the excipient in the amorphous state is contained in an amount of at least 20% by weight, based on the total weight of the excipient.

In a preferred aspect of the freeze-dried preparation according to the present invention, the sugar comprises sucrose or lactose.

Furthermore, in the third aspect of the present invention, the second object is achieved by a process for producing a freeze-dried preparation comprising methylcobalamin which comprises the steps of: (1) dissolving the methylcobalamin or a pharmacologically acceptable salt thereof and an excipient in a solvent; and (2) freeze-drying the solution so as to produce at least an amorphous state of at least one or more species of the excipient.

In a preferred aspect of the production process according to the present invention, freeze-drying is performed so as to further produce an amorphous state of the methylcobalamin or the pharmacologically acceptable salt thereof in the step (2).

In a preferred aspect of the production process according to the present invention, pre-freezing in the step (2) is performed at a temperature at which the excipient does not crystallize, or below the temperature. In particular, pre-freezing during the freeze-drying process is preferably at a temperature at or below an eutectic point of the excipient.

In a preferred aspect of the production process according to the present invention, the excipient which is in the amorphous state comprises a sugar.

In a preferred aspect of the production process according to the present invention, the freeze-dried preparation further comprises a pH adjuster.

In a preferred aspect of the production process according to the present invention, the freeze-dried preparation further comprises an anti-oxidant.

In a preferred aspect of the production process according to the present invention, when the excipient comprises the sugar, the excipient in the amorphous state is contained in an amount of at least 20% by weight, based on the total weight of the sugar.

In a preferred aspect of the production process according to the present invention, the sugar comprises sucrose or lactose.

Advantageous Effects of the Invention

According to the present invention, it is possible to provide a freeze-dried preparation containing methylcobalamin which is highly stable over time. Moreover, according to the production process according to the present invention, it is possible to provide a production process for the freeze-dried preparation comprising methylcobalamin which allows methylcobalamin to be stable in a freeze-dried solid.

Brief Description of Drawings

Figure 1 shows the mixing ratios of sucrose and mannitol used in producing a methylcobalamin freeze-dried preparation according to one embodiment of the present invention.
Figure 2 shows the mixing ratios of sucrose and mannitol used in producing a methylcobalamin freeze-dried preparation according to one embodiment of the present invention.
Figure 3 shows the mixing ratios of lactose and mannitol used in producing a methylcobalamin freeze-dried preparation according to one embodiment of the present invention.
Figure 4 shows the mixing ratios of lactose and mannitol used in producing a methylcobalamin freeze-dried preparation according to one embodiment of the present invention.
Figure 5 shows results for residual ratios of methylcobalamin versus the mixing ratios of sucrose and mannitol as excipients in freeze-dried preparations according to the present invention. In both Figure 5 and Figures 6 through 8 below, 1 M indicates a storage period of 1 month.
Figure 6 shows results for residual ratios of methylcobalamin versus the mixing ratios of sucrose and mannitol as excipients in freeze-dried preparations according to the present invention.
Figure 7 shows results for residual ratios of methylcobalamin versus the mixing ratios of lactose and mannitol as excipients in freeze-dried preparations according to the present invention.
Figure 8 shows results for residual ratios of methylcobalamin versus the mixing ratios of lactose and mannitol as excipients in freeze-dried preparations according to the present invention.
Figure 9 shows results for storage stability at 40°C of freeze-dried preparations according to the present invention in Example 1 through 5 and Comparative Example 1.
Figure 10 shows results for storage stability at 40°C of freeze-dried preparations according to the present invention in Examples 6 through 10 and Comparative Example 2.
Figure 11 shows results for storage stability at 40°C of freeze-dried preparations according to the present invention in Examples 11 and 12 and Comparative Example 3.
Figure 12 shows results for storage stability at 40°C of freeze-dried preparations according to the present invention in Examples 13 and 14 and Comparative Example 4.
Figure 13 shows the x-ray diffraction patterns of freeze-dried preparations containing methylcobalamin manufactured according to the present invention. The x-ray diffraction patterns are shown in Figure 13 when the excipient is (a) sucrose alone (Example 1), (b) a sucrose/mannitol mixture being 1/1 (Example 3), (c) a sucrose/mannitol mixture being 2/3, (d) a sucrose/mannitol mixture being 1/4 (Example 5) and (e) mannitol alone.
Figure 14 shows a composition used to investigate the effect of annealing during the freeze-drying step for a freeze-dried preparation using mannitol.
Figure 15 shows the results for effect on methylcobalamin stability of the presence or absence of annealing during the freeze-drying process for a freeze-dried preparation using mannitol. In this Figure, 3 M indicates 3 months, and the numbers indicate area percentages of methylcobalamin and hydroxycobalamin.

Best Mode for Carrying out the Invention

The present invention provides a freeze-dried preparation containing methylcobalamin which can be used in high-concentration methylcobalamin therapy. The present invention is explained in detail below.

Methylcobalamin used in the present invention is coenzymal vitamin $B_{12}$ type which is present in blood and cerebrospinal fluid. It is more mobile in nerve tissue than other $B_{12}$ homologues. Methylcobalamin has been used in the prevention and treatment of diabetic neural disorders, multiple neuritis and other peripheral nerve disorders, especially for numbness, pain and paralysis, as well as for megaloblastic anemia due to vitamin $B_{12}$ deficiency.

Methylcobalamin is a vitamin $B_{12}$ analogue, and is a compound in which the cyano group bound to the cobalt of vitamin $B_{12}$ (cyanocobalamin) has been replaced with a methyl group. It is a red powder with a molecular weight of 1344.4 and the chemical formula $C_{63}H_{91}CoN_{13}O_{14}P$ (see Formula I below).

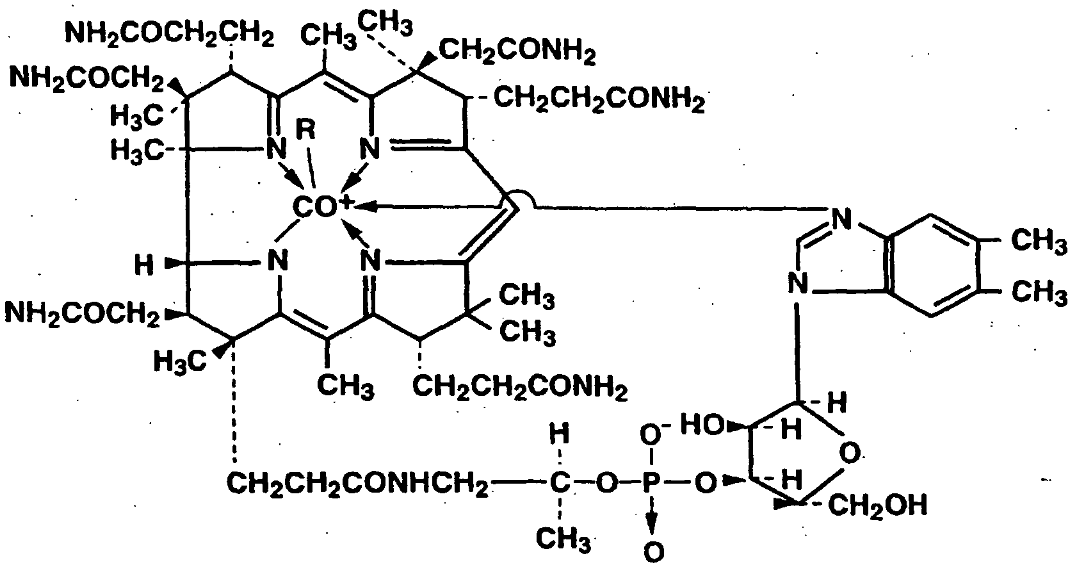

**R=CN : CYANCOBALAMIN (II)**
**R=$CH_3$: METHYLCOBALAMIN (I)**

Methylcobalamin used in the present invention can be produced for example by the method described in Patent Publication S45-38059 via a reduction reaction from cyanocobalamin (Formula II above) followed by methylation. Commercially available raw methylcobalamin of drug grade can also be used. This is sold for example by Sigma Co., Eisai Co. Ltd. and the like. Moreover, a pharmacologically acceptable salt of methylcobalamin may also be used in the present invention. In addition, methylcobalamin may also be an optically active form or a raceme.

Examples of the pharmacologically acceptable salt of methylcobalamin commonly include salts with acids such as inorganic salts of hydrochloric acid, hydrobromic acid, sulfuric acid, nitric acid, phosphoric acid and the like and salts with organic acids such as trifluoroacetic acid, tartaric acid, citric acid, fumaric acid, maleic acid, toluenesulfonic acid, methanesulfonic acid and the like.

The freeze-dried preparation according to the present invention has a high content of methylcobalamin or a pharmacologically acceptable salt thereof as an active ingredient. The term "high content" used in the present invention generally means a content of from 5 to 200 mg, preferably from 10 to 100 mg, more preferably from 25 to 50 mg per 1 vial, or means that a concentration when reconstituted at use is generally from 5 to 50 mg/ml, preferably from 5 to 25 mg/ml, more preferably from 10 to 15 mg/ml per 1 vial.

The freeze-dried preparation according to the present invention comprises at least methylcobalamin and an excipient, and the mixing ratio thereof is from 0 to 50 parts, preferably from 2.5 to 25 parts, more preferably from 5 to 10 parts by weight of the excipient, based on 1 part by weight of methylcobalamin.

The excipient used in the present invention is selected from the sugars. As described below, the excipient can contain at least one or more types of sugar which produce an amorphous state when freeze-dried. Examples of sugar which produce an amorphous state when freeze-dried include such monosaccharides as glucose, fructose and the like and such disaccharides as maltose, lactose, sucrose, trehalose and the like. Lactose and sucrose are particularly desirable as excipients which produce an amorphous state when freeze-dried.

When the excipient used in the present invention is made up of a sugar it is desirable from the standpoint of storage stability of the freeze-dried preparation produced by the production process described below that the excipient in the amorphous state be contained in an amount of at least 20%, preferably 40%, more preferably 50% by weight based on the total weight of sugar.

The freeze-dried preparation according to the present invention may also comprise a pH adjuster. The pH adjuster used may be any which is commonly used in the drug preparations. Examples of the pH adjusters include inorganic acids, inorganic bases, organic bases and buffers. Specific examples include hydrochloric acid, sodium carbonate, sodium bicarbonate, citric acid, sodium citrate, sodium dihydrogen citrate, phosphoric acid, sodium dihydrogenphosphate, sodium hydrogenphosphate, sodium hydroxide, lactic acid, sodium lactate, acetic acid, sodium acetate, or

meglumine.

In addition, the freeze-dried preparation according to the present invention may also comprise an anti-oxidant. The anti-oxidant may be any which is commonly used in the drug preparations, and specific examples include sodium nitrite, ascorbic acid, citric acid, tocophenol, tocophenol acetate, dibutylhydroxytoluene, natural vitamin E, sodium hydrogen sulfite, alphathioglycerin, cysteine hydrochloride or sodium thioglycolate.

Various additives can also be added to increase the solubility of the freeze-dried preparation according to the present invention or adjust its osmotic pressure as an injection. There are no particular limitations on these additives as long as they are in the added amounts of substances generally used as additives in the preparations, and for example the following additives may be added as necessary.

Polyethylene glycol, glycerin are examples of solubilizers for increasing the solubility of the freeze-dried preparation according to the present invention or increasing the dissolution speed, while examples of surfactants include polysorbate, polyoxyethylene polyoxypropylene glycol, polyethylene hydrogenated castor oil, sorbitan sesquioleate and the like. Osmotic pressure adjusters for the freeze-dried preparation according to the present invention include glucose, xylitol, and sorbitol.

In addition, preservatives, stabilizers and other additives which can be used in the pharmaceutical preparations can be added as necessary. Specific examples include benzoic acid or ascorbic acid, and amounts of the additives which can be used by the person skilled in the art may be added.

Next, described is the production process for a freeze-dried preparation according to the present invention. For the freeze-dried preparation according to the present invention, for example a solution of methylcobalamin, an excipient and a pH adjuster which are dissolved in water or in a suitable aqueous solvent (such as a mixture of water and alcohol) is sterilized by filtration by means of a membrane filter as desired. Next, the sterile solution is poured into vials or trays and preferably solidified by ordinary freeze drying. By adopting filter sterilization it is possible to prevent the production of decomposition products which result from heat sterilization, and obtain a preparation with few impurities.

With the freeze-dried preparation according to the present invention produced as described above, it is possible to control decomposition, degeneration and the like of methylcobalamin in the longer term and keep it stable.

In preparing the solution, the methylcobalamin, the excipient and the pH adjuster can be dissolved in water or in an aqueous solvent (such as a mixture of water and alcohol) in accordance with the known methods. The order of dissolving is not dependent on the type of components. The concentration of methylcobalamin in the aqueous liquid is generally from 0.5 mg/ml to 50 mg/ml, preferably from 1 mg/ml to 10 mg/ml, more preferably from 2.5 mg/ml to 7.5 mg/ml. The concentration of the excipient is generally from 0 mg/ml to 200 mg/ml, preferably from 12.5 mg/ml to 100 mg/ml, more preferably from 25 mg/ml to 50 mg/ml.

The pH of the aqueous liquid is generally from 4 to 8, preferably from 6 to 8, more preferably from 6.5 to 7.5. The desired pH can be achieved by appropriate addition of the pH adjuster.

The aqueous liquid prepared in this way is first preferably pre-frozen at or below the eutectic point, then held in vacuum in a drying chamber as the temperature is raised gradually from shelf temperature to the primary drying temperature, and primary drying is performed at this temperature. After primary drying the shelf temperature is raised to the secondary drying temperature, and secondary drying is performed at that temperature. Primary and secondary drying can also be performed at the same temperature.

In a specific process of producing the freeze-dried preparation, a solution containing a predetermined concentration of methylcobalamin and excipient is first sterilized. Predetermined quantities of the solution are then poured into vials, pre-frozen under conditions of from about -60°C to -20°C, and freeze dried by primary drying under a reduced pressure at from about -20 to 10°C and secondary drying under a reduced pressure at from about 15 to 40°C. Generally, nitrogen gas is substituted in the vials, which are stoppered to obtain the freeze-dried preparation.

The eutectic point varies depending on the mixing ratio of components constituting the excipient, but when sugars are generally included, pre-freezing should be performed under temperature conditions of -40°C or less when 1) sucrose and/or lactose and 2) mannitol are both present.

When at least the excipient is present in an amorphous state in the freeze-dried preparation according to the present invention obtained in this way, methylcobalamin can be maintained stably over the long term.

The freeze-dried preparation according to the present invention can be re-dissolved with great ease by adding it to any suitable diluent (liquid for redissolution) to reconstitute the solution before freeze-drying. Examples of such diluents include distilled water for injection, saline and other ordinary infusions (such as glucose, amino acid infusions and the like).

A reconstituted solution of the freeze-dried preparation according to the present invention can be administered parenterally as an intravenous injection, subcutaneous injection, intramuscular injection, drip injection or other injection or as eye drops. This liquid dissolved in injectable distilled water or an infusion is sufficiently stable. In this case the concentration of methylcobalamin in the solution is generally from about 5 mg/ml to 50 mg/ml, preferably from 5 mg/ml to 25 mg/ml, more preferably from 10 mg/ml to 15 mg/ml. The concentration of the excipient is generally from 0 mg/ml to 100 mg/ml, preferably from 12.5 mg/ml to 75 mg/ml, more preferably from 25 mg/ml to 50 mg/ml.

Described are the characteristics of the freeze-dried preparation according to the present invention. The term "stability" used in the present invention refers to chemical and physical stability. A residual ratio of methylcobalamin as an active ingredient will decrease due to degradation over time when stored under heated conditions, and the appearance of the freeze-dried preparation as a preparation will discolor markedly. It is not desirable to use a preparation which exhibits marked coloring and degradation because of the high probability that cloudiness and precipitation will occur during dissolution. In view of this, the residual ratio is generally the marker for stability.

In the present invention, the residual ratio is defined as follows: The freeze-dried preparation containing methylcobalamin is sealed in a vial, and stored under temperature conditions chosen from the range of from 5°C to 40°C. After storage for a constant time at a constant temperature, the concentration of methylcobalamin which is the active ingredient is calculated from the area of the peak corresponding to methylcobalamin in high performance liquid chromatography (hereinafter, "HPLC"). The residual ratio can then be estimated using the formula defined in the example given below from the area of the HPLC peak for methylcobalamin at the beginning of storage and the area of the HPLC peak for methylcobalamin after a predetermined period of storage.

In the freeze-dried preparation according to the present invention, the residual ratio should be 95% or more after no less than 2 years' storage at room temperature. The residual ratio should also be 95% or more after 6 months' storage at 40°C.

The present invention will be explained in more detail using the following examples.

## Examples

### Production Example 1

As one embodiment of the production process for a freeze-dried preparation according to the present invention, stock liquids were prepared from methylcobalamin together with excipients containing a mixture of sucrose and mannitol based on the mixing ratios described below, and then were freeze-dried by the following methods.

The stock liquids were prepared which were a mixture of methylcobalamin (trade name Methycobal, Eisai, 0.5% w/v) and excipient mixtures (2.5% w/v), then were sterilized by filtration, and packed in accurate amounts in vials under sterile conditions and freeze dried. After freeze drying the vials were completely stoppered to produce freeze-dried preparations containing methylcobalamin according to the present invention.

Specifically, amounts of sucrose and mannitol corresponding to the mixing ratios shown in Figure 1 were placed in 100 ml beakers, and 80 ml of distilled water for injection was added thereto followed by stirring to dissolve the excipient by means of a magnetic stirrer. After it was confirmed that the excipient was dissolved adequately, 500 mg of methylcobalamin was added thereto followed by stirring. After it was confirmed that methylcobalamin was dissolved adequately, distilled water was added into 100 ml measuring flasks so that the volume of the solutions was 100 ml.

Next, 18 ml vials were filled with 5 ml of each of the solutions, and freeze drying was performed by pre-freezing at -55°C for 3 hours, primary drying at -10°C for 24 hours (0.1 mbar) and secondary drying at 25°C for 36 hours (0.02 mbar) to obtain freeze-dried preparations according to the present invention, as the following Examples 1 through 5.

Note that a freeze-dried preparation containing only mannitol shown in Figure 1 as the excipient was prepared by the same methods as Comparative Example 1.

### Production Example 2

0.5% w/v methylcobalamin (trade name Methycobal, Eisai K.K.) was mixed with 5.0% w/v of excipient mixtures to prepare stock liquids which were sterilized by filtration, packed in accurate amounts in separate vials under sterile conditions and then freeze dried. After freeze drying, the vials were completely stoppered to produce freeze-dried preparations containing methylcobalamin according to the present invention.

Specifically, amounts of sucrose and mannitol corresponding to the mixing ratios shown in Figure 2 were placed in 100 ml beakers, and 80 ml of injectable distilled water was added thereto followed by stirring to dissolve the excipient by means of a magnetic stirrer. After it was confirmed that the excipient was dissolved adequately, 500 mg of methylcobalamin was added thereto followed by stirring. After it was confirmed that methylcobalamin was dissolved adequately, distilled water was added in 100 ml measuring flasks so that the volume of the solutions was 100 ml.

Next, 18 ml vials were filled with 5 ml of each of the solutions, and freeze drying was performed by pre-freezing at -55°C for 3 hours, primary drying at -10°C for 24 hours (0.1 mbar) and secondary drying at 25°C for 36 hours (0.02 mbar) to obtain freeze-dried preparations according to the present invention, as the following Examples 6 through 10.

A freeze-dried preparation containing only mannitol shown in Figure 2 as the excipient was prepared by the same methods as Comparative Example 2.

Production Example 3

Next, stock liquids were prepared using methylcobalamin together with mixtures of lactose and mannitol as the excipients based on the mixing ratios shown in Figure 3 according to the methods described in Production Example 1 (using lactose and mannitol in place of sucrose and mannitol), and freeze-dried preparations were produced under the same conditions as in Production Example 1 (Examples 11 and 12).

A freeze-dried preparation containing only mannitol shown in Figure 3 as the excipient was prepared by the same methods as Comparative Example 3.

Production Example 4

Next, stock liquids were prepared by use of methylcobalamin together with mixtures of lactose and mannitol as the excipients based on the mixing ratios shown in Figure 4 according to the methods described in Production Example 1 (using lactose and mannitol in place of sucrose and mannitol), and freeze-dried preparations were produced under the same conditions as in Production Example 1 (Examples 13 and 14).

A freeze-dried preparation containing only mannitol shown in Figure 4 as the excipient was prepared by the same methods as Comparative Example 4.

Production Example 5

12.5 g of sucrose was measured into a 100 ml beaker and dissolved in 80 ml of previously-prepared pH 7.0 1 mM phosphoric acid buffer. After it was confirmed that the sucrose was dissolved sufficiently, 0.5 g of methylcobalamin was added to the buffer solution. After dissolution was confirmed, the buffer solution was prepared to be 100 ml with distilled water in a 100 ml measuring flask. 15 ml vials were filled with 5 ml each of the solution prepared in this way, which was then freeze dried under the same conditions as in Production Example 1 to obtain a freeze-dried preparation containing methylcobalamin (Example 15).

Production Example 6

25 g of lactose was measured into a 1000 ml beaker and dissolved in 800 ml of water for injection. 0.69 g of sodium dihydrogen phosphate monohydrate was then added thereto and stirred thoroughly. After it was confirmed that the lactose and sodium dihydrogen phosphate were dissolved adequately, 5 g of methylcobalamin was added thereto and methylcobalamin remaining in the weighed container was washed with the water for methylcobalamin injection. After dissolution was confirmed, the pH was adjusted to 7.0 by addition of a suitable amount of previous-prepared 0.1 M aqueous sodium hydroxide solution. The solution was adjusted to be 1000 ml with distilled water in a 1000 ml measuring cylinder. 15 ml vials were filled with 5 ml each of the solution prepared in this way, which was then freeze dried under the same conditions as in Production Example 1 to obtain a freeze-dried preparation containing methylcobalamin (Example 16).

Production Example 7

12.5 g of lactose was measured into a 100 ml beaker and 1 g of citric acid monohydrate and 80 ml of water for injection were added and stirred. After it was confirmed that the lactose and citric acid were dissolved, 0.5 g of methylcobalamin was added thereto. After dissolution was confirmed, the pH was adjusted to be 7.0 by addition of a suitable amount of previous-prepared 0.1 M aqueous sodium hydroxide solution and the solution was adjusted to be 100 ml with distilled water in a 100 ml measuring flask. 15 ml vials were filled with 5 ml each of the solution prepared in this way, which was then freeze dried under the same conditions as in Production Example 1 to obtain a freeze-dried preparation containing methylcobalamin (Example 17).

Production Example 8

12.5 g each of sucrose and lactose were measured into separate 100 ml beakers, and dissolved in 80 ml of previously-prepared pH 7.0 1 mM phosphoric acid buffer. After confirmation of dissolution, 0.5 g of methylcobalamin was added to each solution and dissolved by stirring. After confirmation of dissolution, the solutions were adjusted to be 100 ml with distilled water in 100 ml measurement flasks. 15 ml vials were filled with 15 ml each of the solutions prepared in this way, which were then freeze dried under production conditions of 3 hours' pre-freezing at -55°C, 24 hours' primary drying at -10°C (0.1 mbar) and 36 hours' secondary drying at 25°C (0.002 mbar) to obtain freeze-dried preparations containing methylcobalamin (Examples 18 and 19).

[Evaluation of storage stability]

Storage stability was evaluated as follows based on a residual ratio of methylcobalamin.
That is, the freeze-dried preparations containing methylcobalamin which had been prepared under sterile conditions in accordance with the production examples were stored for 1 month in a thermostatic chamber at 5°C, 25°C and 40°C. The stored freeze-dried preparations were dissolved in a mobile phase during HPLC measurement, and sample solutions were prepared using 25 ml measurement flasks. Using these samples, methylcobalamin content (the residual ratio) was measured by the following test methods to evaluate methylcobalamin storage stability.
Methylcobalamin content was measured by HPLC analysis under the following conditions.

HPLC conditions

Column: hydrosphere C18, S-5 μm, 250 x 4.6 mm I.D.
Mobile phase: acetonitrile/0.02 mol/L phosphoric acid buffer (pH 3.5)/1-sodium hexanesulfonate (2/8/3.8, v/v/w)

HPLC analysis conditions

1) column temperature: 40°C, 2) sample temperature: 8°C, 3) flow rate: 1 mL/min,
4) detector: ultraviolet spectroscopy (measurement wavelength: 266 nm)

The residual ratio of methylcobalamin was calculated from area of the peak corresponding to methylcobalamin by HPLC. The residual ratio was derived as follows from a ratio of methylcobalamin peak area before start of storage to methylcobalamin peak area after storage:

Residual ratio (%) = {(methylcobalamin peak area after storage)/(total peak area after storage)/{(methylcobalamin peak area before storage)/(total peak area before storage)} x 100.

Total peak area here means the total area of all peaks measured by HPLC. The results for the residual ratio of Production Examples 1 through 4 obtained by these methods are shown in Figures 5 through 8, respectively.
Moreover, the results for the residual ratio (%) of methylcobalamin when each example and comparative example was stored for 1 month and/or 3 months in a thermostatic chamber at 40°C are shown in Figures 9 through 12.

[Powder x-ray diffraction]

The freeze-dried preparations containing methylcobalamin obtained in Production Example 1 were subjected to powder x-ray diffraction. The crystal conditions of the preparations before storage and after storage for 1 month in a thermostatic chamber at 5°C, 25°C and 40°C were observed under the following conditions.
The measurement conditions for powder x-ray diffraction were as follows.

X-ray: Cu K-alpha 1/30 kV/40 mA
Goniometer: RINT2000 vertical goniometer
Filter: Kβ filter
Divergence slit: 1/2 deg
Scattering slit: 1/2 deg
Receiving slit: 0.15 mm
Scanning speed: continuous
Scan speed: 2°/min
Scan width: 0.02°
Scanning axis: 2θ/θ
Scanning range: 5-40°
Offset: 0°

Fixed angle: 0°

The typical x-ray diffraction patterns of the preparations of Production Example 1 before storage are shown in Figure 13. In Figure 13, (a), (b), (c), (d) and (e) indicate the x-ray diffraction patterns for Example 1, Examples 3 through 5 and Comparative Example 1 in Production Example 1, respectively. No differences in x-ray diffraction patterns before and after analysis were observed under any of the storage conditions.

As can be seen from the results in Figures 5 and 6, it was shown that regardless of the amount of excipient mixed with the methylcobalamin, when the proportion of sucrose in the sucrose/mannitol excipient mixture is 20% or more, the residual ratio of methylcobalamin in freeze-dried compositions containing methylcobalamin is extremely high, and storage stability of methylcobalamin is excellent.

On the other hand, as can be seen from the x-ray diffraction patterns in Figure 13, there was almost no x-ray diffraction peak for (a) of Example 1, which contained methylcobalamin and sucrose. Taking the content of methylcobalamin in the freeze-dried preparation into consideration, this means that the sucrose was in an amorphous state. As the proportion of mannitol in the excipient mixture increases, however, a diffraction peak derived from mannitol appears.

Accordingly, from the results for the residual ratio and x-ray diffraction pattern, it was demonstrated that when sucrose constitutes 20% or more of the excipient, or in other words when at least one of the excipients is amorphous, a freeze-dried preparation containing methylcobalamin has excellent storage stability.

Note that the storage stability of the samples from Examples 15 through 19 was evaluated under conditions of 60°C for one month, and all samples were extremely stable, with no decrease observed in the residual ration of methylcobalamin.

Figures 7 and 8 show results for the residual ratio of methylcobalamin based on the mixing ratios of lactose and mannitol in the excipient mixture. It was clear from the results in Figures 7 and 8 that the residual ratio of methylcobalamin is very high when the proportion of lactose in an amorphous state in a lactose/mannitol excipient mixture is 50% or more regardless of how much of the excipient mixture is added to the methylcobalamin.

Moreover, from the results shown in Figures 9 and 10 for the residual ratio of methylcobalamin after 3 months' storage at 40°C, it was clear that when the proportion of sucrose in a sucrose-mannitol excipient mixture for methylcobalamin is 20% or more, the residual ratio of methylcobalamin in a freeze-dried preparation containing methylcobalamin is extremely high.

Furthermore, from the results shown in Figure 11 for the residual ratio of methylcobalamin after 1 month's storage at 40°C and the results shown in Figure 12 for the residual ratio of methylcobalamin after 3 months' storage at 40°C, it was shown that when the proportion of lactose in a lactose-mannitol excipient mixture for methylcobalamin is 50% or more, the residual ratio of methylcobalamin in a freeze-dried preparation containing methylcobalamin is extremely high, and storage stability is excellent.

In view of the above, it was thus demonstrated that with the freeze-dried preparation containing methylcobalamin of the present invention, stability of methylcobalamin is greater when one or more of the excipients is in an amorphous state. The storage stability of the freeze-dried preparation containing methylcobalamin is also improved by inclusion of pH adjusters and anti-oxidants.

However, it is known that crystallization of mannitol is promoted by heat treatment (also called annealing) during pre-freezing in the freeze-drying process for preparations to which mannitol is added as an excipient. See for example Izutsu, K., Yoshioka, S. and Terao, T., Pharma. Res. 10, 1232-1237; Pikal, M.J., Dellerman, K.M., Roy, M.L. and Riggin, R.M., Pharma. Res. 8 (1991) 427-436; Izutsu, K., Ocheda, S., Aoyagi, N. and Kojima, S., Int. J. Pharm. 273 (2004) 85-93; Wu, S., Leung, D., Tretyakov, L., Hu, J., Guzzetta, A. and Wang, J., Int. J. Pharm. 200 (200) 1-16; and Yoshinari, T., Forbes, R., York, R. and Kawashima, Y., Int. J. Pharm. 258 (2003) 109-120. This has been mainly studied to evaluate the effect of freeze-drying conditions on quality for protein preparations. According to the report of Izutsu et al, since the exothermic peak for crystallization of mannitol occurs at around -23°C in DSC measurement, crystallization of mannitol can be promoted by performing heat treatment above this temperature in the pre-freeze-drying process.

Therefore, as shown below, the stability of freeze-dried preparations containing methylcobalamin according to the present invention was studied with and without annealing to promote crystallization of mannitol.

Figure 14 shows a composition which was used to investigate the effects of heat treatment in a freeze-dried preparation using mannitol. The freeze-drying conditions with and without an annealing step were as follows.

With annealing step

(1) pre-freezing for 2.5 hours at -55°C, (2) temperature raised over 3 hours, (3) annealing for 3 hours at -10°C, (4) temperature lowered over 3 hours, (5) primary drying for 2.5 hours at -55°C (-10°C, 24 hours (0.1 mbar)).

Without annealing step

(A) pre-freezing for 2.5 hours at -55°C, followed by (B) primary drying (-10°C, 24 hours (0.1 mbar)).

[Evaluation of storage stability]

Storage stability was evaluated as in the previous examples based on changes in methylcobalamin content according to HPLC after 3 months' storage in a thermostatic chamber at various temperatures.

Figure 15 shows results for stability of methylcobalamin in the freeze-dried preparation using mannitol with and without heat treatment in the freeze-drying process. The figures for methylcobalamin (B12-CH3) and hydroxycobalamin (B12-OH) in Figure 15 were calculated from the HPCL measurements as (peak area of B12-CH3 or B12-OH)/total of all peak areas) x 100.

The results shown in Figure 15 indicate that when freeze drying includes an annealing step which promotes crystallization of mannitol, not only is methylcobalamin stability lower than if there were no annealing but there is increased production of hydroxycobalamin, a degradation product of methylcobalamin. This is evidence that methylcobalamin is more unstable in a crystalline environment than in a non-crystalline environment.

With the freeze-dried preparation containing methylcobalamin according to the present invention, storage stability of methylcobalamin is improved by suppressing the crystalline state of the excipient, and it is possible to provide a freeze-dried preparation on reconstitution at use which is suitable for dissolving in solution for preparing injections.

## Claims

**1.** A freeze-dried preparation comprising methylcobalamin or a pharmacologically acceptable salt thereof and an excipient,
wherein said excipient comprises at least one sugar, said sugar being selected from the group consisting of glucose, fructose, maltose, lactose, sucrose and trehalose, wherein said sugar in said excipient is in an amorphous state.

**2.** The freeze-dried preparation according to claim 1, wherein said sugar that is present in said amorphous state is present in said excipient in an amount that is at least 20% by weight , based on the total weight of said excipient.

**3.** The freeze-dried preparation according to claim 1, wherein the methylcobalamin or the pharmacologically acceptable salt thereof is also in an amorphous state.

**4.** The freeze-dried preparation according to any one of claim 1 or claim 2, further comprising a pH adjuster.

**5.** The freeze-dried preparation according to any one of claim 1 or claim 2, further comprising an anti-oxidant.

## Patentansprüche

**1.** Gefriergetrocknete Zubereitung, umfassend Methylcobalamin oder ein pharmakologisch akzeptables Salz davon und einen Hilfsstoff, wobei der Hilfsstoff mindestens einen Zucker umfasst, wobei der Zucker aus der Gruppe, bestehend aus Glucose, Fructose, Maltose, Lactose, Saccharose und Trehalose ausgewählt ist, wobei der Zucker in dem Hilfsstoff in einem amorphen Zustand ist.

**2.** Gefriergetrocknete Zubereitung nach Anspruch 1, wobei der Zucker, der in dem amorphen Zustand vorliegt, in dem Hilfsstoff in einer Menge vorhanden ist, die mindestens 20 Gewichts-% ist, bezogen auf das Gesamtgewicht des Hilfsstoffs.

**3.** Gefriergetrocknete Zubereitung nach Anspruch 1, wobei das Methylcobalamin oder das pharmakologisch akzeptable Salz davon auch in einem amorphen Zustand ist.

**4.** Gefriergetrocknete Zubereitung nach Anspruch 1 oder Anspruch 2, weiterhin umfassend einen pH-Regulator.

**5.** Gefriergetrocknete Zubereitung nach Anspruch 1 oder Anspruch 2, weiterhin umfassend ein Antioxidans.

## Revendications

1. Préparation lyophilisée comprenant de la méthylcobalamine ou un sel pharmacologiquement acceptable de celle-ci et un excipient,
dans laquelle ledit excipient comprend au moins du sucre, ledit sucre étant sélectionné du groupe constitué de glucose, de fructose, de maltose, de lactose, de sucrose et de tréhalose, où ledit sucre dans ledit excipient se trouve dans un état amorphe.

2. Préparation lyophilisée selon la revendication 1, dans laquelle ledit sucre qui est présent dans ledit état amorphe est présent dans ledit excipient en une proportion qui est d'au moins 20% en poids, sur la base du poids total dudit excipient.

3. Préparation lyophilisée selon la revendication 1, dans laquelle la méthylcobalamine pu le sel pharmacologiquement acceptable de celle-ci se trouve également dans un état amorphe.

4. Préparation lyophilisée selon l'une quelconque des revendications 1 ou 2, comprenant en outre un régulateur de pH.

5. Préparation lyophilisée selon l'une quelconque des revendications 1 ou 2, comprenant en outre un antioxydant.

# FIG.1

| SAMPLE | SUCROSE | MANNITOL |
|---|---|---|
| EXAMPLE 1 | 2.5g | 0g |
| EXAMPLE 2 | 1.5g | 1.0g |
| EXAMPLE 3 | 1.25g | 1.25g |
| EXAMPLE 4 | 1.0g | 1.5g |
| EXAMPLE 5 | 0.5g | 2.0g |
| COMPARATIVE EXAMPLE 1 | 0g | 2.5g |

# FIG.2

| SAMPLE | SUCROSE | MANNITOL |
|---|---|---|
| EXAMPLE 6 | 5.0g | 0g |
| EXAMPLE 7 | 3.0g | 2.0g |
| EXAMPLE 8 | 2.5g | 2.5g |
| EXAMPLE 9 | 2.0g | 3.0g |
| EXAMPLE 10 | 1.0g | 4.0g |
| COMPARATIVE EXAMPLE 2 | 0g | 5.0g |

# FIG.3

| SAMPLE | SUCROSE | MANNITOL |
|---|---|---|
| EXAMPLE 11 | 2.5g | 0g |
| EXAMPLE 12 | 1.25g | 1.25g |
| COMPARATIVE EXAMPLE 3 | 0g | 2.5g |

# FIG.4

| SAMPLE | SUCROSE | MANNITOL |
|---|---|---|
| EXAMPLE 13 | 5.0g | 0g |
| EXAMPLE 14 | 2.5g | 2.5g |
| COMPARATIVE EXAMPLE 4 | 0g | 5.0g |

# FIG.5

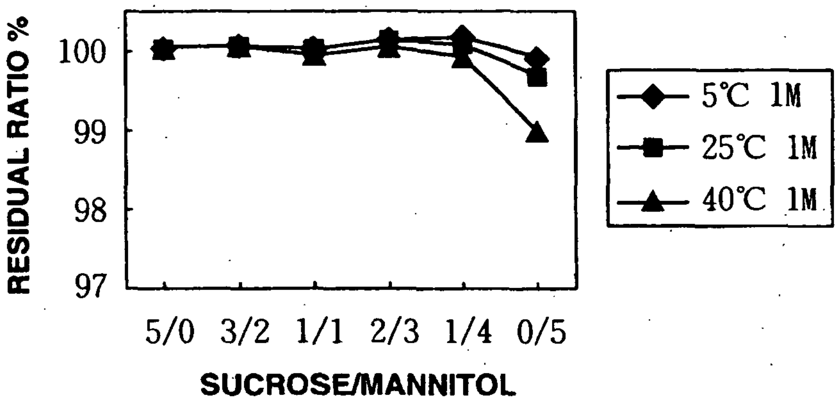
RESIDUAL RATIO %
100
99
98
97
5/0 3/2 1/1 2/3 1/4 0/5
SUCROSE/MANNITOL
5℃ 1M
25℃ 1M
40℃ 1M

# FIG.6

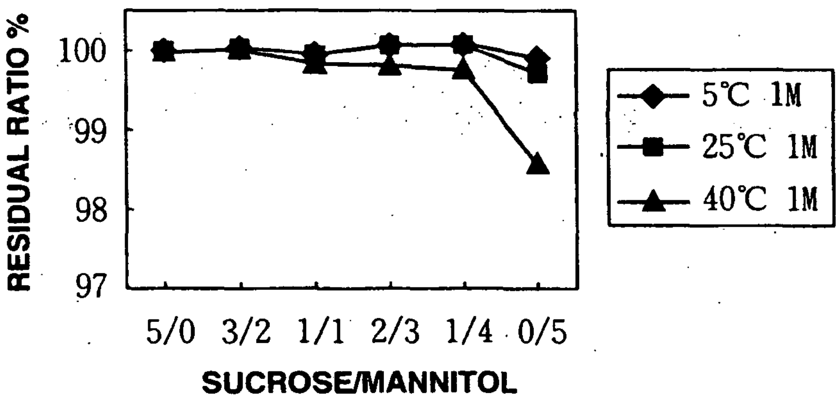
RESIDUAL RATIO %
100
99
98
97
5/0 3/2 1/1 2/3 1/4 0/5
SUCROSE/MANNITOL
5℃ 1M
25℃ 1M
40℃ 1M

# FIG.7

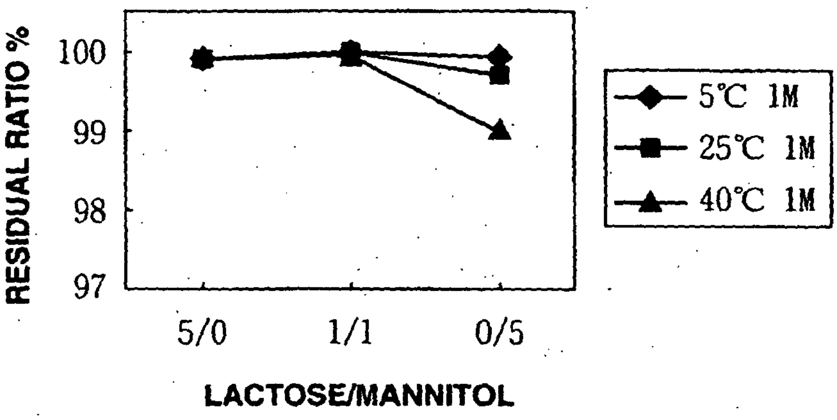
RESIDUAL RATIO %
100
99
98
97
5/0
1/1
0/5
LACTOSE/MANNITOL
5℃ 1M
25℃ 1M
40℃ 1M

# FIG.8

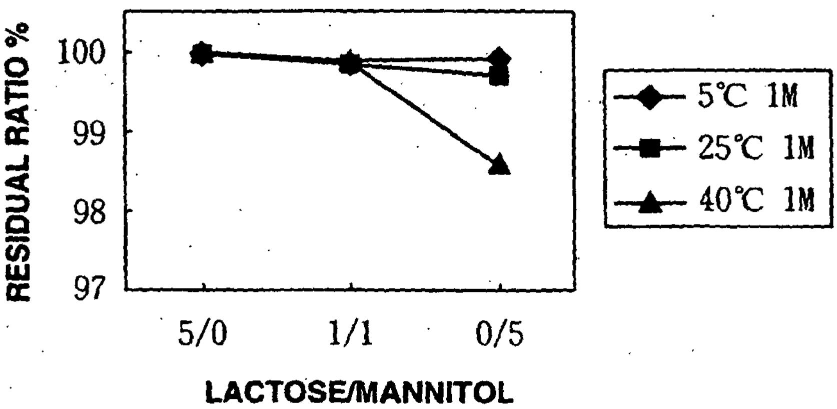
RESIDUAL RATIO %
100
99
98
97
5/0
1/1
0/5
LACTOSE/MANNITOL
5℃ 1M
25℃ 1M
40℃ 1M

FIG.9

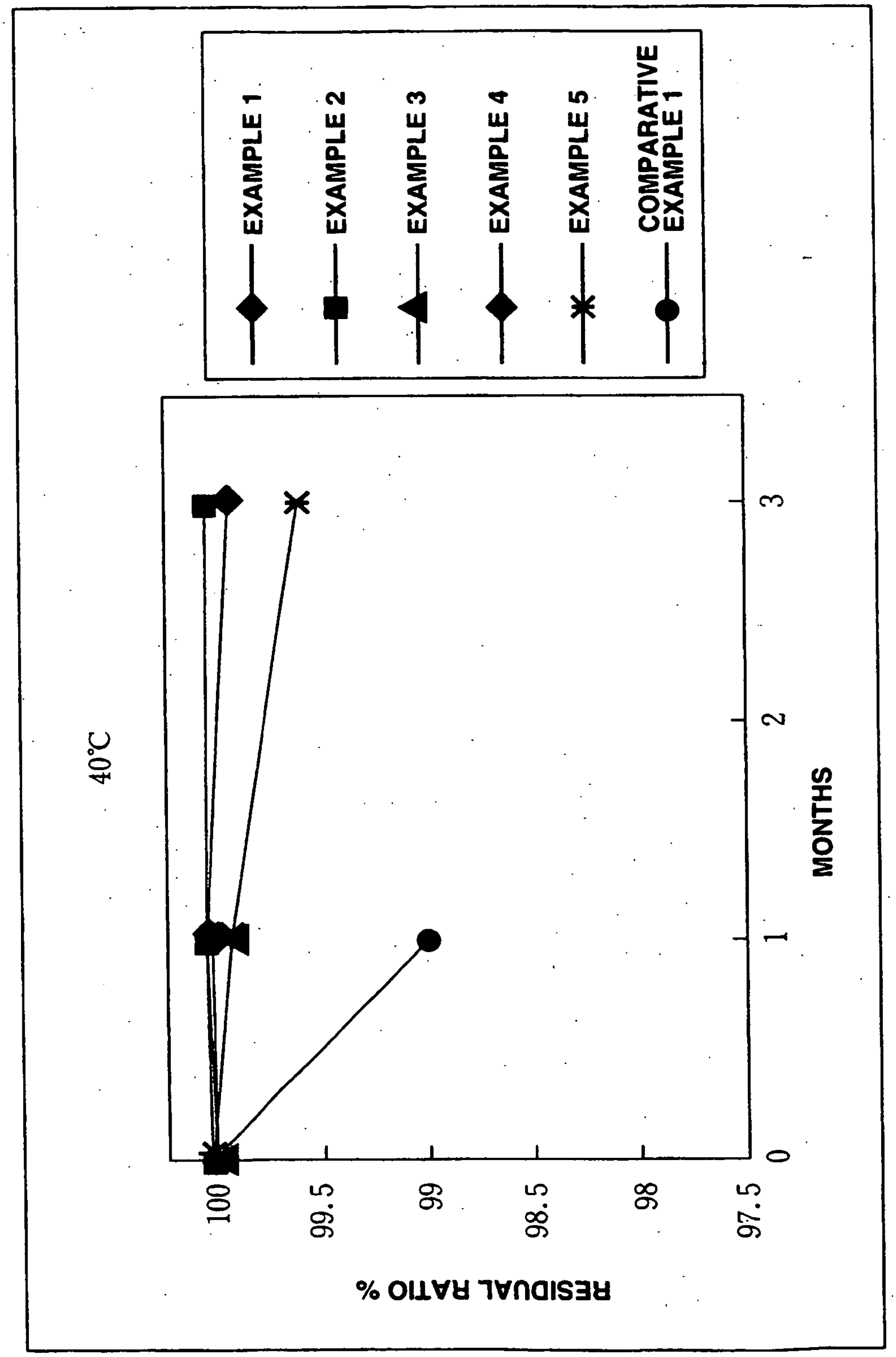
40℃
RESIDUAL RATIO %
100
99.5
99
98.5
98
97.5
0
1
2
3
MONTHS
EXAMPLE 1
EXAMPLE 2
EXAMPLE 3
EXAMPLE 4
EXAMPLE 5
COMPARATIVE EXAMPLE 1

FIG.10

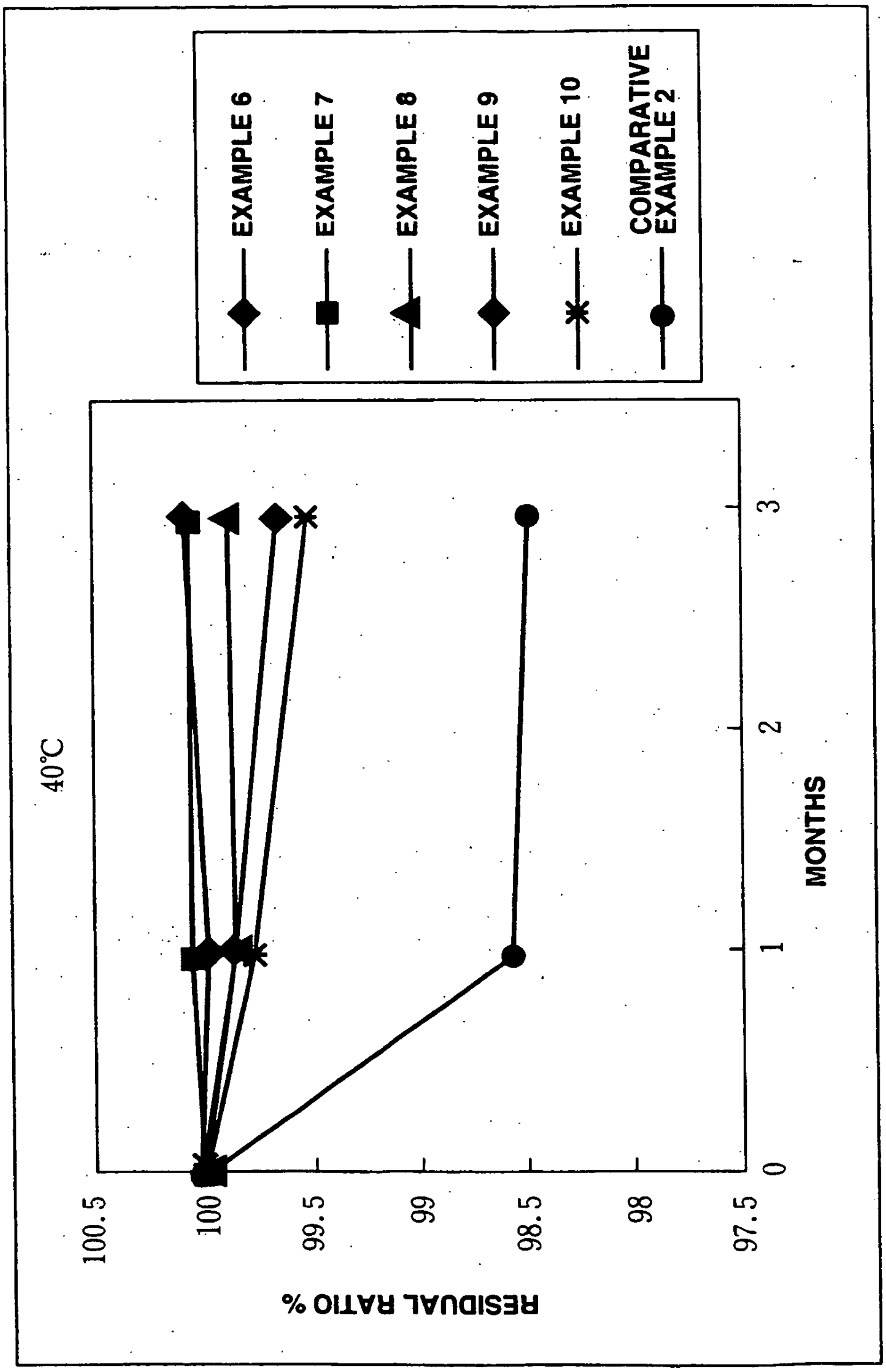
40°C
RESIDUAL RATIO %
100.5
100
99.5
99
98.5
98
97.5
0
1
2
3
MONTHS
EXAMPLE 6
EXAMPLE 7
EXAMPLE 8
EXAMPLE 9
EXAMPLE 10
COMPARATIVE EXAMPLE 2

FIG.11

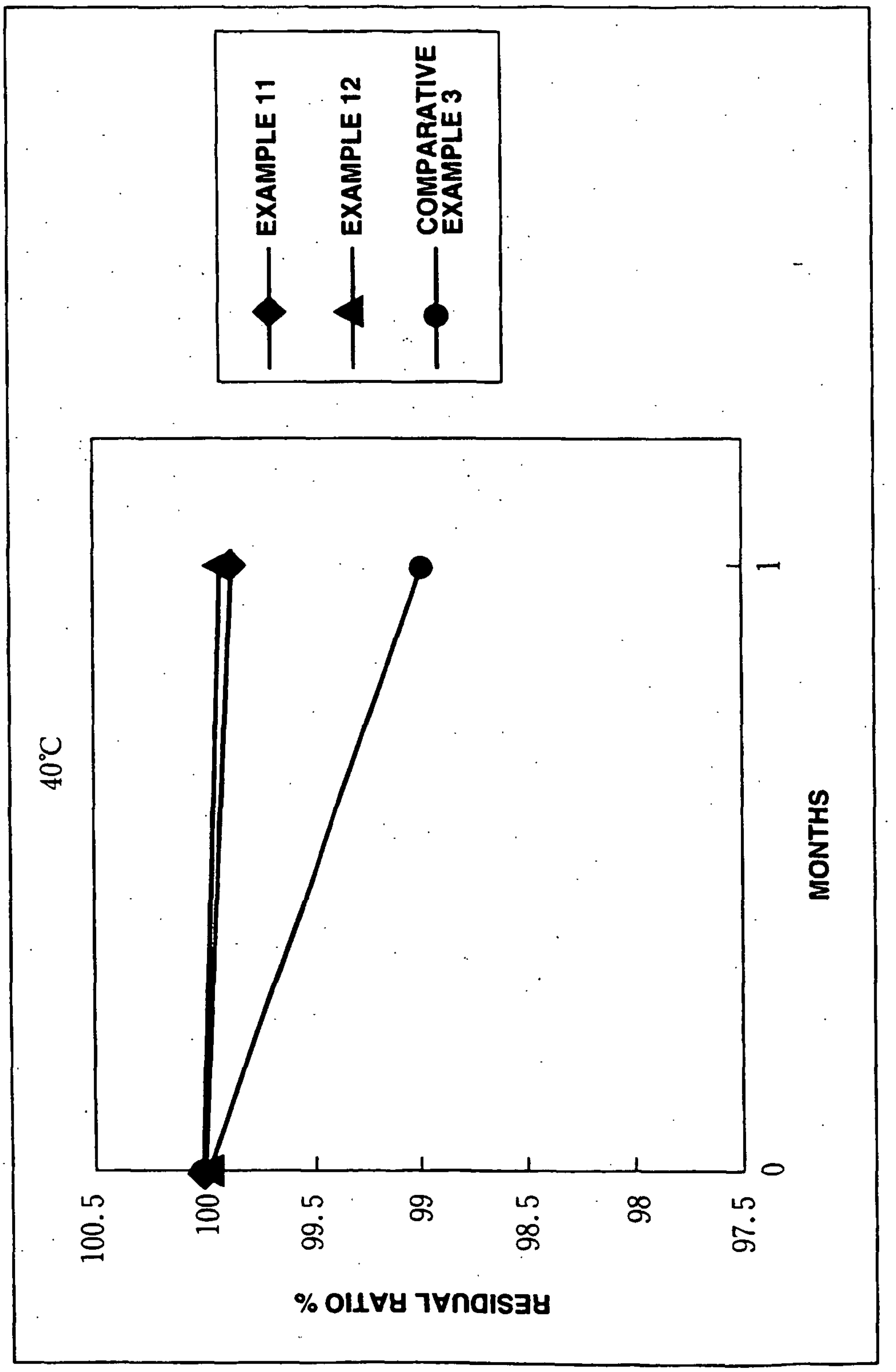
40℃
EXAMPLE 11
EXAMPLE 12
COMPARATIVE EXAMPLE 3
RESIDUAL RATIO %
100.5
100
99.5
99
98.5
98
97.5
0
1
MONTHS

FIG.12

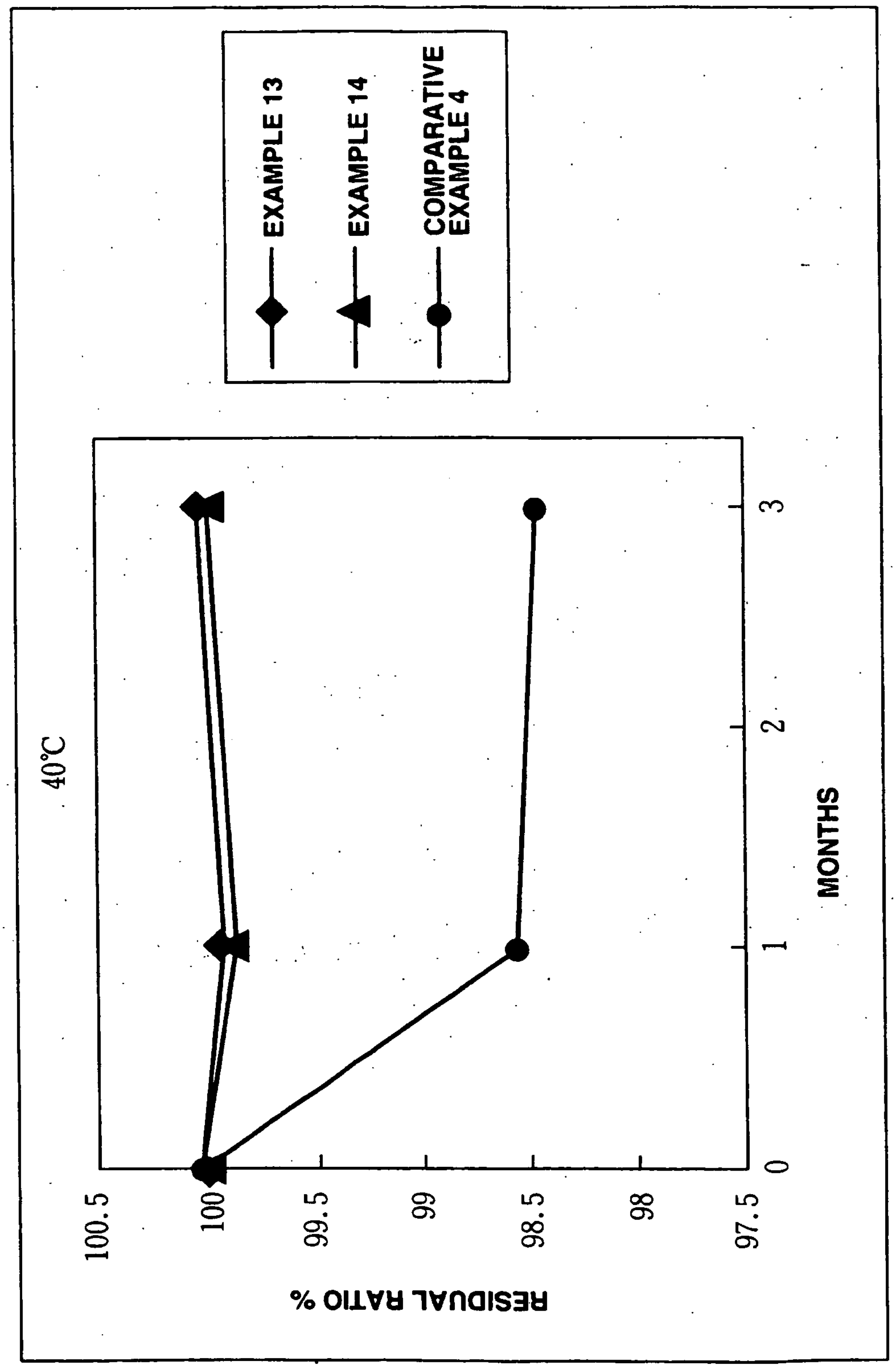
40°C
RESIDUAL RATIO %
100.5
100
99.5
99
98.5
98
97.5
0
1
2
3
MONTHS
EXAMPLE 13
EXAMPLE 14
COMPARATIVE EXAMPLE 4

FIG.13

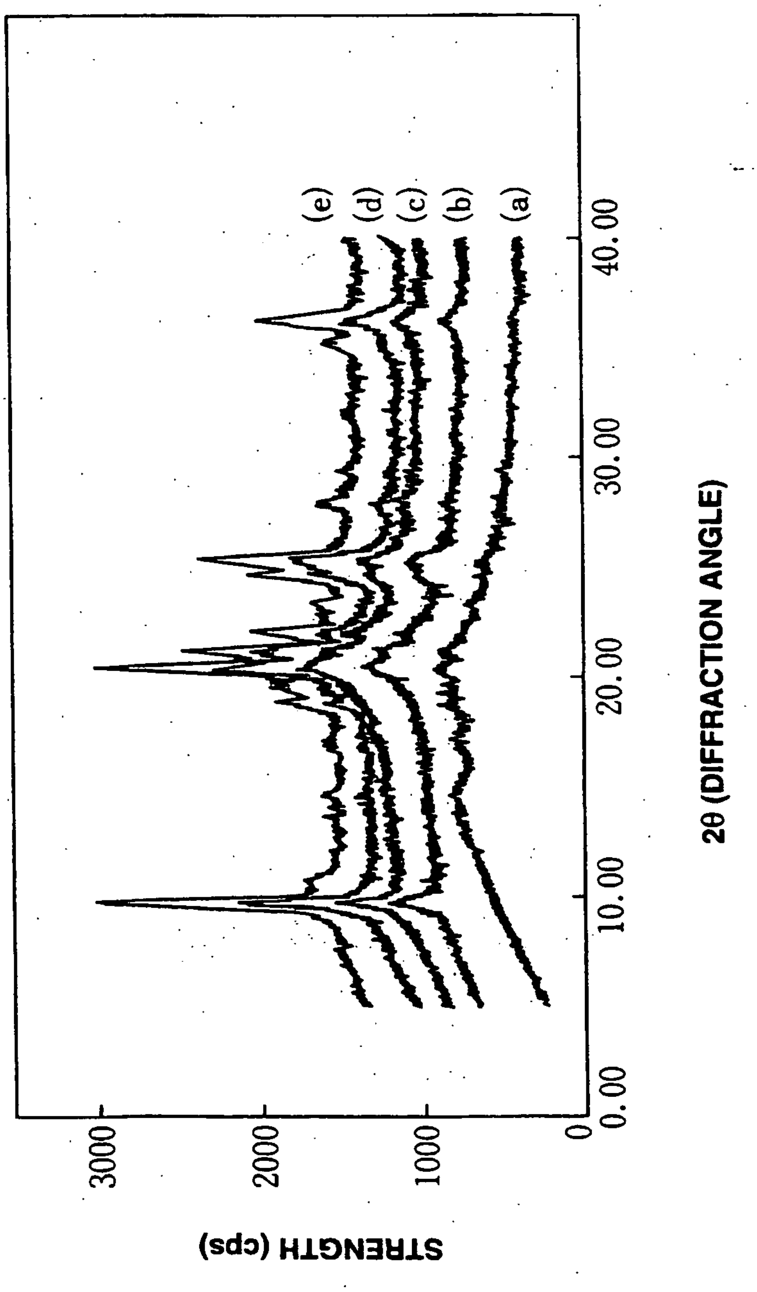
STRENGTH (cps)
3000
2000
1000
0
0.00
10.00
20.00
30.00
40.00
2θ (DIFFRACTION ANGLE)
(e)
(d)
(c)
(b)
(a)

# FIG.14

| | AMOUNT |
|---|---|
| METHYLCOBALAMIN | 25mg |
| MANNITOL | 125mg |
| PURIFIED WATER FOR INJECTION | TO TOTAL OF 5mL |

# FIG.15

| | INITIAL | | 5℃ 3M | | 25℃ 3M | | 40℃ 3M | |
|---|---|---|---|---|---|---|---|---|
| | B12-CH3 | B12-OH | B12-CH3 | B12-OH | B12-CH3 | B12-OH | B12-CH3 | B12-OH |
| WITH ANNEALING | 99.80 | 0.15 | 99.71 | 0.23 | 99.33 | 0.57 | 98.17 | 1.39 |
| WITHOUT ANNEALING | 99.74 | 0.21 | 99.82 | 0.18 | 99.59 | 0.34 | 99.21 | 0.63 |

B12-CH3 : METHYLCOBALAMIN
B12-OH : HYDROXYCOBALAMIN

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H10218775 B
- JP S6238 A
- JP S633137 B
- JP 132514 H


### Non-patent literature cited in the description


- **Izutsu, K. ; Yoshioka, S. ; Terao, T.** *Pharma. Res.,* vol. 10, 1232-1237
- **Pikal, M.J. ; Dellerman, K.M. ; Roy, M.L. ; Riggin, R.M.** *Pharma. Res.,* 1991, vol. 8, 427-436
- **Izutsu, K. ; Ocheda, S. ; Aoyagi, N. ; Kojima, S.** *Int. J. Pharm.,* 2004, vol. 273, 85-93
- **Wu, S. ; Leung, D. ; Tretyakov, L. ; Hu, J. ; Guzzetta, A. ; Wang, J.** *Int. J. Pharm.,* vol. 200 (200), 1-16
- **Yoshinari, T. ; Forbes, R. ; York, R ; Kawashima, Y.** *Int. J. Pharm.,* 2003, vol. 258, 109-120